# EUROPÄISCHE PATENTSCHRIFT

(11) **EP 0 700 397 B1**
(45) Veröffentlichungstag und Bekanntmachung des Hinweises auf die Patenterteilung: **04.09.2002**
(21) Anmeldenummer: 94918342.0
(22) Anmeldetag: 19.05.1994
(51) Int. Cl.: C07D 417/12, A61K 31/425, C07D 417/14, C07D 277/34, C07D 417/10

(54) **NEUE THIAZOLIDINDIONE UND DIESE ENTHALTENDE ARZNEIMITTEL**
NEW THIAZOLIDINDIONES AND DRUGS CONTAINING THEM
NOUVELLES THIAZOLIDINEDIONES ET MEDICAMENTS CONTENANT CES SUBSTANCES

(30) Priorität: 25.05.1993 DE 4317320
(43) Veröffentlichungstag der Anmeldung: 13.03.1996
(73) Patentinhaber: Roche Diagnostics GmbH, 68298 Mannheim (DE)
(72) Erfinder: MERTENS, Alfred, D-69198 Schriesheim (DE); WOLFF, Hans-Peter, D-68167 Mannheim (DE); FREUND, Peter, D-68775 Ketsch (DE)
(74) Vertreter: Braun, Axel
(86) Internationale Anmeldenummer: EP9401619
(87) Internationale Veröffentlichungsnummer: WO94027995

(56) Entgegenhaltungen:
- EP-A- 0 177 353
- EP-A- 0 207 605
- EP-A- 0 299 620
- EP-A- 0 559 571
- WO-A-89/08652

## Beschreibung

Gegenstand der vorliegenden Erfindung sind Thiazolidindione, Verfahren zu deren Herstellung und Arzneimittel, die diese Verbindungen enthalten.

Die Erfindung betrifft Thiazolidindione der allgemeinen Formel I 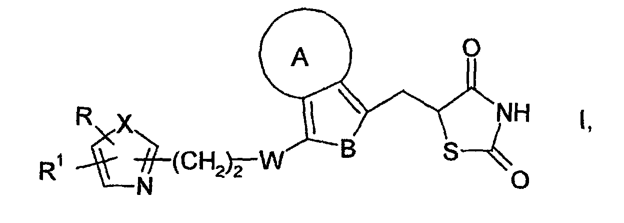 in der
- A: einen carbocyclischen Ring mit 5 oder 6 Kohlenstoffatomen oder einen heterocyclischen Fünf- oder Sechsring mit 1 oder 2 Heteroatomen darstellt, wobei die Heteroatome gleich oder verschieden sein können und Sauerstoff, Stickstoff oder Schwefel bedeuten, und die Heterocyclen gegebenenfalls an einem oder mehreren Stickstoffatomen ein Sauerstoffatom tragen können,
- B: -CH=CH-, -N=CH- oder -CH=N-,
- W: CH₂, O, CH(OH) oder CO,
- x: S, O oder NH,
- R: Naphthyl, Pyridyl, Furyl, Thienyl oder Phenyl, das gegebenenfalls mit C₁-C₃ Alkyl, CF₃, C₁-C₃ Alkoxy, F, Cl oder Brom mono- oder disubstituiert ist, und
- R1: Wasserstoff oder C₁-C₆ Alkyl
bedeutet, sowie deren Tautomere, Enantiomere, Diastereomere und physiologisch verträgliche Salze.

In der Literatur werden bereits ähnliche Verbindungen mit anti-diabetischer Wirkung erwähnt. So werden im US Patent 4617312 Thiazolidindione mit hypoglykämischer Wirkung beschrieben, wobei in ortho Stellung zum Thiazolidindion ein Alkoxyrest zwingend erforderlich ist. In Chem. Pharm. Bull., 30, 3563, 1982 wird die Synthese von 5-[4-(-2-methyl-2-phenyl-propoxy)benzyl]thiazolidin-2,4-dionen und ihre antidiabetische Wirkung vorgestellt. Die US Patente 4340605, 4725610 und die europäische Anmeldung EP-A-389699 umfassen 4-Alkoxybenzylthiazolidindione, die im Alkylteil durch einen Heterocyclus substituiert sind, mit hypoglykämischer Wirkung. Die europäische Anmeldung EP-A-332332 beansprucht ebenfalls eine antidiabetische Wirkung für Benzylthiazolidindione, die in para-Stellung durch verschiedene Reste substituiert sein können. Das US Patent 4703052 beschreibt Derivate als Antidiabetika, die mit einem Bicyclus verknüpft sind, wobei jedoch der aromatische Ring des Bicyclus, der den Thiazolidinrest trägt, keinen weiteren Substituenten enthalten darf. Die europäischen Patentanmeidungen EP-A-283035 und EP-A-299620 umfassen Benzoxazol- und Benzofuran-verknüpfte Thiazolidindione mit antidiabetischer Wirkung. Oxazolyl-alkoxybenzyl-thiazolidindione werden in der europäischen Patentanmeldung EP-A-177358 offenbart.

Es wurde nun überraschend gefunden, daß aromatische Ringe, die im gleichen Ringsystem durch den Thiazolidindionrest und einen weiteren Substituenten substituiert sind und zusätzlich einen aromatischen Fünf- oder Sechsring ankondensiert haben, wertvolle pharmakologische Eigenschaften haben.

Die erfindungsgemäßen Verbindungen eignen sich insbesondere zur Herstellung von Antidiabetica zur oralen Behandlung des Diabetes mellitus, vor allem des Typs II bzw. Typs IIb. Hierbei spielt nach derzeitigen Erkenntnissen die Verwertungsstörung von Insulin und Glucose als eine der Hauptursachen des Altersdiabetes eine große Rolle. Durch diese Verwertungsstörung wird eine Hyperinsulinämie hervorgerufen, die wiederum als Risikofaktor für die Entstehung makroangiopathischer Komplikationen gilt. Untersuchungen mit adiposen Typ-II-Diabetikern zeigten, daß sich mit den erfindungsgemäßen Substanzen sowohl die Glucose- als auch die Insulinspiegel senken ließen. Aufgrund ihres besonderen Wirkmechanismus haben die Substanzen weiterhin einige Vorteile: Sie verursachen keine Hypoglykämien und können, da sie auch den Insulinspiegel reduzieren, das Arterioskleroserisiko des Typ-II-Diabetikers senken. Sie eignen sich daher auch zur Prophylaxe vor arteriosklerotischen Erkrankungen. Außerdem besitzen sie einen positiven Einfluß auf erhöhte Blutdruckwerte und bewirken eine Senkung der Triglycerid- und Cholesterinspiegel.

Die Reste für das Ringsystem A sind carbocyclische Ringe mit 5 oder 6 Kohlenstoffatomen oder ein heterocyclischer Fünf- oder Sechsring mit 1 oder 2 Heteroatomen, wobei die Heteroatome gleich oder verschieden sein können und Sauerstoff, Stickstoff oder Schwefel bedeuten.

Die Reste für R sind Naphthyl, Pyridyl, Furyl, Thienyl oder Phenyl, die bevorzugt gegebenenfalls mit Methyl, CF₃, Methoxy, Fluor, Chlor oder Brom mono- oder disubstituiert sind.

Bevorzugte Reste für A sind carbocyclische aromatische Ringe mit 6 Kohlenstoffatomen oder ein heterocyclischer aromatische Fünf- oder Sechsring mit einem Heteroatom, wobei das Heteroatom Sauerstoff, Stickstoff oder Schwefel bedeuten kann. Ganz besonders bevorzugt ist A ein Phenyl- oder Pyridylring.

Als besonders bevorzugt für W gilt O, CH(OH) und CO.

Für X ist die Bedeutung S oder O besonders bevorzugt.

Besonders bevorzugte Reste für R sind Pyridyl, Furyl, Thienyl oder Phenyl, die gegebenenfalls mit Methyl, Methoxy, Fluor oder Chlor mono- oder disubstituiert sind. Hierbei sind Phenyl, Methylphenyl, Methoxyphenyl, Fluorphenyl und Chlorphenyl ganz besonders bevorzugt.

Für R¹ ist Wasserstoff, Methyl oder Ethyl ganz besonders bevorzugt.

Zur Herstellung von Arzneimitteln werden die Verbindungen der allgemeinen Formel I in an sich bekannter Weise mit geeigneten pharmazeutischen Trägersubstanzen, Aroma-, Geschmacks- und Farbstoffen gemischt und beispielsweise als Tabletten oder Dragees ausgeformt oder unter Zugabe entsprechender Hilfsstoffe in Wasser oder Öl, wie z.B. Olivenöl, suspendiert oder gelöst.

Die Substanzen der allgemeinen Formel I können in flüssiger oder fester Form oral und parenteral appliziert werden. Als Injektionsmedium kommt vorzugsweise Wasser zur Anwendung, welches die bei Injektionslösungen üblichen Stabilisierungsmittel, Lösungsvermittler und/oder Puffer enthält. Derartige Zusätze sind z.B. Tartrat- oder Borat-Puffer, Ethanol, Dimethylsulfoxid, Komplexbildner (wie Ethylendiamintetraessigsäure), hochmolekulare Polymere (wie flüssiges Polyethylenoxid) zur Viskositätsregulierung oder Polyethylen-Derivate von Sorbitanhydriden.

Feste Trägerstoffe sind z.B. Stärke, Lactose, Mannit, Methylcelluslose, Talkum, hochdisperse Kieselsäure, höhermolekulare Fettsäuren (wie Stearinsäure), Gelatine, Agar-Agar, Calciumphosphat, Magnesiumstearat, tierische und pflanzliche Fette oder feste hochmolekulare Polymere (wie Polyethylenglykole). Für die orale Applikation geeignete Zubereitungen können gewünschtenfalls Geschmacks- und Süßstoffe enthalten.

Die verabreichte Dosierung hängt vom Alter, der Gesundheit und dem Gewicht des Empfängers, dem Ausmaß der Krankheit, der Art gleichzeitiger gegebenenfalls durchgeführter weiterer Behandlungen, der Häufigkeit der Behandlungen und der Art der gewünschten Wirkung ab. Üblicherweise beträgt die tägliche Dosis der aktiven Verbindung 0.1 bis 50 mg/kg Körpergewicht. Normalerweise sind 0.5 bis 40 und vorzugsweise 1.0 bis 20mg/kg/Tag in einer oder mehreren Anwendungen pro Tag wirksam, um die gewünschten Resultate zu erhalten.

Die erfindungsgemäßen Verbindungen- der allgemeinen Formel I werden nach literaturbekannten Verfahren (J. Med. Chem. 35, 1853, 1992, J. Med. Chem. 35, 2617, 1992, Chem. Pharm. Bull. 30, 3580, 1982, Chem. Pharm. Bull 30, 3563, 1982) hergestellt, indem man
a) Verbindungen der allgemeinen Formel II 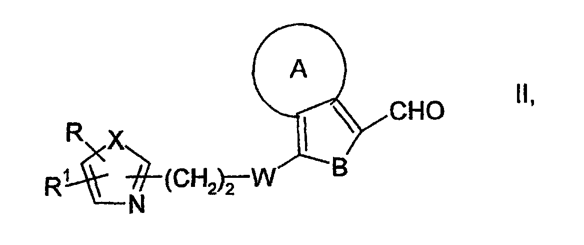 in der A, B, W, X, R und R¹ die oben angegebene Bedeutung haben, mit Thiazolidindion zu Verbindungen der allgemeinen Formel III 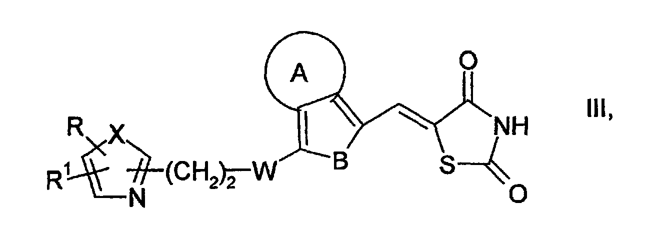 in der A, B, W, X, R und R¹ die oben angegebene Bedeutung haben, umsetzt und anschließend durch Reduktion der Doppelbindung die Verbindungen der allgemeinen Formel 1 erhält, oder 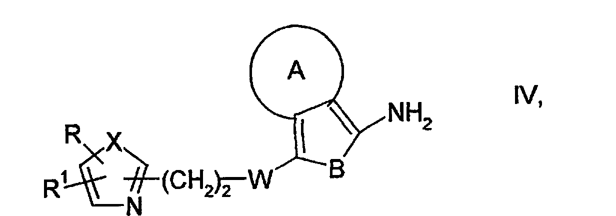
b) Verbindungen der allgemeinen Formel IV
   in der A, B, W, X, R und R¹ die oben angegebene Bedeutung haben, mit NaNO₂ in Gegenwart von Acrylester und HCl oder HBr zu Verbindungen der allgemeinen Formel V 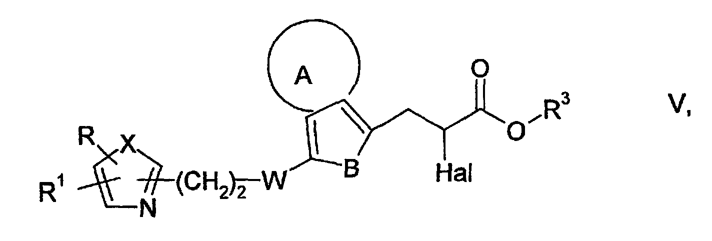 in der A, B, W, X, R und R¹ angegebene Bedeutung haben, Hal für Chlor oder Brom steht und R³ einen C₁-C₆ -Alkylrest bedeutet, umsetzt und anschließend Verbindungen der allgemeinen Formel V mit Thioharnstoff zu Verbindungen der allgemeinen Formel VI 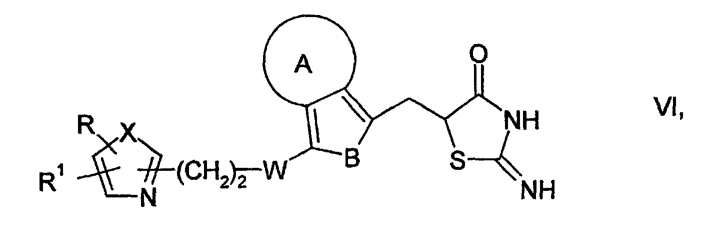 in der A, B, W, X, R und R¹ die angegebenen Bedeutung haben, cyclisiert und durch Behandeln mit Säure in Verbindungen der allgemeinen Formel I umwandelt.

Die Umsetzung von Verbindungen der allgemeinen Formel II mit Thiazolidindion gelingt in polaren und unpolaren Lösungsmitteln gegebenenfalls unter Zusatz einer Hilfsbase wie z.B. Natriumacetat oder Triethylamin bei Temperaturen zwischen -40°C und dem Siedepunkt des gewählten Lösungsmittel. Die anschließende Reduktion der Verbindungen der allgemeinen Formel III wird vorteilhaft mit Wasserstoff in Gegenwart von Metallkatalysatoren wie z.B. Pt oder Pd oder auch durch Homogenkatalyse in inerten Lösungsmitteln bei Temperaturen zwischen -20°C und dem Siedepunkt das Lösungsmittels durchgeführt. Gegebenenfalls kann die katalytische Hydrierung durch Erhöhung des Druckes beschleunigt werden.

Die Umwandlungen von Verbindungen der allgemeinen Formel IV in Verbindungen der allgemeinen Formel V gelingt vorteilhaft in wäßrigen Lösungsmittel mit NaNO₂ in Gegenwart von Säuren wie z.B. Salzsäure und Bromwasserstoffsäure, wobei das intermediär entstandene Diazoniumsalz mit Acrylester-Derivaten gegebenenfalls unter Zusatz von Cu(I)salzen umgesetzt wird.
Diese α-Halogencarbonsäureester lassen sich vorteilhaft mit Thioharnstoff in protischen Lösungsmitteln bei Temperaturen von -20° C bis zum Siedepunkt des Lösungsmittels gegebenenfalls unter Zusatz einer Hilfsbase wie z.B. Natriumacetat oder NEt₃ zu Verbindungen der allgemeinen Formel VI umsetzen, aus denen man durch Hydrolyse unter Zusatz von Säuren wie z.B. Salzsäure oder unter Verwendung einer Lauge wie z.B. Natriumhydroxyd vorteilhaft in einem protischen Lösungsmittel, das gegebenenfalls erhitzt werden kann, die Verbindungen der allgemeinen Formel I erhält.

Zu reinen Enantiomeren der Verbindungen der Formel I kommt man entweder durch Racematspaltung (über Salzbildung mit optisch aktiven Säuren oder Basen), oder indem man in die Synthese optische aktive Ausgangsstoffe einsetzt.

Im Sinne der vorliegenden Erfindung kommen ausser den in den Beispielen genannten Verbindungen und der durch Kombination aller in den Ansprüchen genannten Bedeutungen der Substituenten die folgenden Verbindungen der Formel I in Frage, die als racemische Gemische oder in optisch aktiver Form bzw. als reine R- und S-Enantiomere vorliegen können:
1. 5-[4-[2-(5-Methyl-2-phenyl-4-oxazolyl)ethoxy]-1-naphthylmethyl]-2,4-thiazolidindion
2. 5-[7-[2-(5-Methyl-2-phenyl-4-oxazolyl)ethoxy]-4-indolylmethyl]-2,4-thiazolidindion
3. 5-[7-[2-(5-Methyl-2-phenyl-4-oxazolyl)ethoxy]-4-benzofuranylmethyl]-2,4-thiazolidindion
4. 5-[7-[2-(5-Methyl-2-phenyl-4-oxazolyl)ethoxy]-4-benzothiophenylmethyl]-2,4-hiazolidindion
5. 5-(4-[2-(5-Methyl-2-phenyl-4-oxazolyl)ethoxy]-7-indolylmethyl]-2,4-thiazolidindion
6. 5-[4-[2-(5-Methyl-2-phenyl-4-oxazolyl)ethoxy]-7-benzofuranylmethyl)-2,4-thiazolidindion
7. 5-[4-(2-(5-Methyl-2-phenyl-4-oxazolyl)ethoxy]-7-benzothiophenylmethyl]-2,4-thiazoldindion
8. 5-[8-[2-(5-Methyl-2-phenyl-4-oxazolyl)ethoxy]-5-chinolinylmethyl]-2,4-thiazolidindion
9. 5-[8-[2-(5-Methyl-2-phenyl-4-oxazolyl)ethoxy]-5-isochinolinylmethyl]-2,4-thiazolidindion
10. 5-[5-[2-(5-Methyl-2-phenyl-4-oxazolyl)ethoxy]-8-isochinolinylmethyl]-2,4-thiazolidindion
11. 5-[5-[2-(5-Methyl-2-phenyl-4-oxazolyl)ethoxy]-8-chinolinylmethyl)-2,4-thiazolidindion
12. 5- [1-[2-(5-Methyl-2-phenyl-4-oxazolyl)ethoxy]-4-isochinolinylmethyl]-2,4-thiazolidindion
13. 5-[4-[2-(5-Methyl-2-phenyl-4-oxazolyl)ethoxy]-1-isochinolinylmethyl]-2,4-thiazolidindion
14. 5-[4-[2-[5-Methyl-2-(4-methylphenyl)-4-oxazolyl)ethoxy]-1-naphthylmethyl]-2,4-thiazolidindion
15. 5-[4-[2-[5-Methyl-2-(2-thienyl)-4-oxazolyl)ethoxy]-1-naphthylmethyl]-2,4-thiazolidindion
16. 5-[4-[2-[5-Methyl-2-(4-pyridyl)-4-oxazolyl)ethoxy]-1-naphthylmethyl]-2,4-thiazolidindion
17. 5-[4-(5-Methyl-2-phenyl-4-oxazolyl)methoxy]-1-naphthylmethyl]-2,4-thiazolidindion
18. 5-[4-[3-(5-Methyl-2-phenyl-4-oxazolyl)propionyl]-1-naphthylmethyl]-2,4-thiazolidindion
19. 5-[4-[3-(5-Methyl-2ephenyl-4-oxazolyl)-1-hydroxypropyl]-1-naphthylmethyl]-2,4-thiazolidindion
20. 5-[4-[2-(5-Methyl-2-phenyl-4-oxazolyl)acetyl]-1-naphthylmethyl]-2,4-thiazolidindion
21. 5-[4-[2-(5-Methyl-2-phenyl-4-thiazolyl)ethoxy]-1-naphthylmethyl]-2,4-thiazolidindion
22. 5-[4-(2-(5-Methyl-2-phenyl-4-imidazolyl)ethoxy]-1-naphthylmethyl)- 2,4-thiazolidindion

### BEISPIEL 1

### 5-[4-[2-(5-Methyl-2-phenyl-4-oxazolyl)ethoxy]-1-naphthylmethyl]-2,4-thiazolidindion

a) 8.6 g (0.05 mol) 4-Hydroxynaphthalin-1-aldehyd, 13.07 g (0.05 mol) 5-Methyl-2-phenyl-4-(2-bromethyl)-oxazol und 3.4 g (0.05 mol) NaOEt wurden in 100 ml Ethanol 16 Std. am Rückfluß erhitzt. Anschließend wurde eingeengt, der Rückstand in CH₂Cl₂ aufgenommen, getrocknet und eingeengt. Nach Kristallisation aus Isopropanol erhält man 5.2 g 4-[2-(5-Methyl-2-phenyl-4-oxazolyl)ethoxy]naphthalin-1-aldehyd vom Schmp. 130-133°C.
b) 5.07 g (0.014 mol) der vorangegangenen Verbindung, 3.87 g (0.042 mol) Thiazolidindion und 0.28 ml Piperidin werden in 150 ml Ethanol 8 Std. zum Rückfluß erhitzt. Nach dem Abkühlen wurde der Niederschlag durch Absaugen isoliert, mit Ether gewaschen und mit 50 ml Eisessig kurz auf 50°C erwärmt. Nach Zufügen von 100 ml Ether wurde erneut abgesaugt und der Rückstand mit Ether gewaschen. Man erhält 3.28 g 5-[4-[2-(5-Methyl-2- phenyl-4-oxazolyl)ethoxy]naphthyl]methylen]-2,4-thiazolidindion vom Smp. 248-250°C.
c) 456 mg der vorgenannten Verbindung wurden in 40 ml THF in Gegenwart von 200 mg Pd/C (10 %) in 36 Std. bei 50° C und 6bar katalytisch hydriert. Nach Abtrennen des Katalysators und Einengen des Lösungsmittels erhält man nach Kristallisation aus Ethanol 265 mg der Titelverbindung vom Smp. 188-191°C.

### BEISPIEL 2

a) Analog Beispiel 1 erhält man ausgehend von 5-Methyl-2-(4-pyridyl)-4- (2-bromethyl)oxazol die Titelverbindung 5-[4-[2-[5-Methyl-2-(4-pyridyl)-4- oxazolyl]ethoxy]-1-naphthylmethyl]-2,4-thiazolidindion vom Smp. 238°C (Zers.).
b) Analog Beispiel 1 erhält man ausgehend von 5-Methyl-2-(2-thienyl)-4- (2-bromethyl)oxazol die Titelverbindung 5-[4-[2-[5-Methyl-2-(2-thienyl)-4- oxazolyl]ethoxy]-1-naphthylmethyl]-2,4-thiazoldindion vom Smp. 159-162°C.

### BEISPIEL 3

### 5-[4-[2-(5-Methyl-2-phenyl-4-oxazolyl)ethoxy]-7-benzothiophenmethyl]-2,4-thiazolidindion

a) 5.15 g (0.034 mol) 4-Hydroxybenzothiophen wurden in 130 ml Methylethylketon gelöst und mit 9.4 g (0.068 mol) K₂CO₃ und 20 g (0.068 mol) 5-Methyl-2- phenyl-4-(2-bromethyl)oxazol versetzt. Der Ansatz wurde 72 Std. unter Rückfluß gekocht, eingedampft, mit Essigester aufgenommen und dreimal mit 2 N NaOH ausgeschüttelt. Nach Kohlen und Eindampfen der organischen Phase wurde aus Essigester/Isohexan kristallisiert. Man erhält 8.8 g 4-[2-(5-Methyl-2-phenyl-4-oxazolyl)ethoxy]benzothiophen vom Smp. 130-132°C.
b) 10 g (30 mmol) der vorangegangenen Verbindung wurde in 30 ml Eisessig mit 1.3 ml (3 mmol) 100proz. HNO₃ unter Kühlung nitriert. Nach 1 Std. bei 25°C wurde Wasser zugesetzt, mit Essigester extrahiert, eingedampft und der Rückstand über Kieselgel (Laufmittel: Heptan/Methylethylketon 4:1) chromatographisch gereinigt. Man erhält 4.1 g 4-[2-(5-Methyl-2-phenyl-4-oxazolyl)-ethoxy]-7-nitrobenzothiophen vom Smp. 148-149°C.
C) 3.1 g (8.06 mmol) der vorangegangenen Verbindung wurden in 150 ml THF mit 0.6 g Pd/C 10% hydriert. Nach Entfernen des Katalysators und Abdampfen des Lösungsmittels erhält man 2.8 g 4-[2-(5-Methyl-2-phenyl-4-oxazolyl)- ethoxy]-7-aminobenzothiophen> das ohne weitere Reinigung weiterverarbeitet wurde.
d) 2.85 g (8.2 mmol) der vorangegangenen Verbindung wurde in 80 ml Aceton und 3 ml 48proz. HBr suspendiert. In diese Suspension wurde bei 0°C 0.55 g NaNO₂ in 4 ml Wasser zugetropft. Nach 15 Minuten wurden 10.3 ml Acrylsäuremethylester zugetropft und anschließend bei 10°C 20 mg CuBr zugegeben. Der Ansatz erwärmt sich auf 30°C und wird noch 1 Std. bei dieser Temperatur gehalten. Anschließend wird eingedampft, in Essigester aufgenommen, mit Wasser gewaschen, gekohlt und erneut eingedampft. Der Rückstand wurde über Kieselgel (Laufmittel: Heptan/Methylethylketon 4:1) chromatographisch gereinigt. Man erhält 1.2 g 3-[4-[2-(5-Methyl-2-phenyl-4- oxazolyl)ethoxy]benzothiophen-7-yl]-2-brompropionsäuremethylester vom Smp. 99-100°C.
e) 1 g (2 mmol) der vorangegangenen Verbindung wurden in 25 ml Ethanol mit 0.23 g Thioharnstoff und 0.16 g Natriumacetat 6 Std. am Rückfluß gekocht. Anschließend wurde eingedampft und der Rückstand mit Wasser/Ether/ lsohexan versetzt und abgesaugt. Der feste Rückstand wurde anschließend mit 20 ml 2 N HCl und 30 ml Ethylenglykolmonoethylether 5 Std. am Rückfluß gekocht. Nach Eindampfen wurde mit Bicarbonatlösung versetzt, der Niederschlag abgesaugt und mit Essigester verrieben. Man erhält 0.6 g der Titelverbindung vom Smp. 200-202°C.

### BEISPIEL 4

### Pharmakologische Versuchsbeschreibung

Die nachfolgend beschriebenen Untersuchungen wurden an ob/ob-Mäusen durchgerührt. Die ob/ob-Maus ist ein Modell mit den Charakteristika: hyperphagie, Hyperglykämie, Hyperinsulinämie und periphere Insulin-Resistenz. Dieses Modell eignet sich deshalb besonders zur Abprüfung von Substanzen, die eine Wirkung auf die periphere Insulin-Resistenz besitzen, welche nach heutiger wissenschaftlicher Auffassung ursächlich an der Entstehung eines Typ II Diabetes beteiligt ist.

In dem oben genannten Modell wurden die Verbindungen der Beispiele 1, 2 a, 2 b und 3 geprüft. Hierzu wurden gefütterte ob/ob-Mäuse mit 100 mg/kg mit der jeweiligen Substanz täglich p.o. über 5 Tage hinweg behandelt, sowie eine Kontrollgruppe lediglich mit dem Lösungsvermittler Methyl-Cellulose. Am 5. Tag wurden die Tiere getötet und mit dem gewonnenen Blut die Blutglucose-Konzentration sowie die Insulin-Konzentration bestimmt. Die Blutglucose-Konzentration wurde unter Zuhilfenahme eines EPOS-Analyzer 5060®, Eppendorf Gerätebau, Hamburg, mittels der kinetischen Hexokinase-Methode (Schmidt, F.H., Klin. Wschr. 39, 1244, 1961) bestimmt. Die Insulin-Konzentration wurde mit einem Radio-Immuno-Assay (Pharmacia Insulin-RIA 100) von Pharmacia Diagnostics AB Uppsala, Schweden, bestimmt.
Die Ergebnisse sind der beigefügten Tabelle zu entnehmen. Blood glucose _{End K} und Insulin _{End K} stellen die Werte das mitgeführten Kontrollkollektivs nach 5 Tagen dar, die Spalten Blood glucose und Insulin repräsentieren die mit den Substanzen erhaltenen Werte. Deutlich ist die Blutglucose- und Insulin-senkende Wirkung der Verbindungen der Beispiele 1, 2 a, 2 b und 3 zu erkennen.

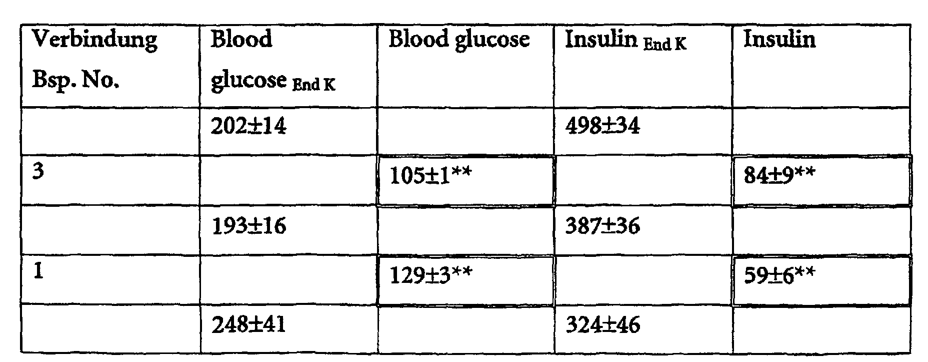

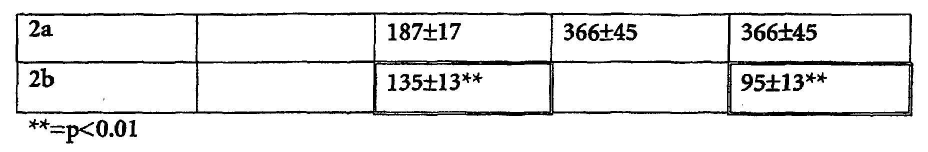

## Patentansprüche

1. Verbindungen der Formel 1 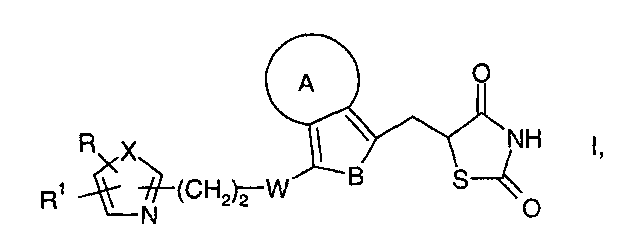 in der
A einen carbocyclischen Ring mit 5 oder 6 Kohlenstoffatornen oder einen heterocyclischen Fünf- oder Sechsring mit 1 oder 2 Heteroatomen darstellt, wobei die Heteroatome Sauerstoff, Stickstoff oder Schwefel bedeuten, und die Heterocyclen gegebenenfalls an einem oder mehreren Stickstoffatomen ein Sauerstoffatom tragen können,
B -CH=CH-, -N=CH- oder -CH=N-,
W CH₂, O, CH(OH) oder CO,
X S, O oder NH,
R Naphthyl, Pyridyl, Furyl, Thienyl oder Phenyl, das gegebenenfalls mit C₁-C₃ Alkyl, CF₃, C₁-C₃ Alkoxy, F, Cl oder Brom mono- oder disubstituiert ist, und
R¹ Wasserstoff oder C₁-C₆ Alkyl
bedeutet, sowie deren Tautomere, Enantiomere, Diastereomere und physiologisch verträgliche Salze.

2. Verbindung ausgewählt aus der Gruppe
a) 5-[4-[2-(5-Methyl-2-phenyl-4-oxazolyl)ethoxy]-1-naphthylmethyl]-2,4-thiazolidindion
b) 5-[7-[2-(5-Methyl-2-phenyl-4-oxazolyl)ethoxy]-4-indolylmethyl]-2,4-thiazolidindion
c) 5-[7-[2-(5-Methyl-2-phenyl-4-oxazolyl)ethoxy]-4-benzofuranylmethyl]-2,4-thiazolidindion
d) 5- [7- [2-(5-Methyl-2-phenyl-4-oxazolyl)ethoxy]-4-benzothiophenylmethyl]-2,4-hiazolidindion
e) 5-(4-[2-(5-Methyl-2-phenyl-4-oxazolyl)ethoxy]-7-indolylmethyl]-2,4-thiazolidindion
f) 5-[4-[2-(5-Methyl-2-phenyl-4-oxazolyl)ethoxy]-7-benzofuranylmethyl)-2,4-thiazolidindion
g) 5-[4-(2-(5-Methyl-2-phenyl-4-oxazolyl)ethoxy]-7-benzothiophenylmethyl]-2,4-thiazoldindion
h) 5-[8-[2-(5-Methyl-2-phenyl-4-oxazolyl)ethoxy]-5-chinolinylmethyl]-2,4-thiazolidindion
i) 5-[8-[2-(5-Methyl-2-phenyl-4-oxazolyl)ethoxy]-5-isochinolinylmethyl]-2,4-thiazolidindion
j) 5-[5-[2-(5-Methyl-2-phenyl-4-oxazolyl)ethoxy]-8-isochinolinylmethyl]-2,4-thiazolidindion
k) 5-[5-[2-(5-Methyl-2-phenyl-4-oxazolyl)ethoxy]-8-chinolinylmethyl)-2,4-thiazolidindion
l) 5-[1-[2-(5-Methyl-2-phenyl-4-oxazolyl)ethoxy]-4-isochinolinylmethyl]-2,4-thiazolidindion
m) 5-[4- [2- (5-Methyl-2-phenyl-4-oxazolyl)ethoxy]-1-isochinolinylmethyl]-2,4-thiazolidindion
n) 5-[4-[2-[5-Methyl-2-(4-methylphenyl)-4-oxazolyl)ethoxy]-1-naphthylmethyl]-2,4-thiazolidindion
o) 5-[4-[2-[5-Methyl-2-(2-thienyl)-4-oxazolyl)ethoxy]-1-naphthylmethyl]-2,4-thiazolidindion
p) 5-[4-[2-[5-Methyl-2-(4-pyridyl)-4-oxazolyl)ethoxy]-1-naphthylmethyl]-2,4-thiazolidindion
q) 5-[4-(5-Methyl-2-phenyl-4-oxazolyl)methoxy]-1-naphthylmethyl]-2,4-thiazolidindion
r) 5-[4-[3-(5-Methyl-2-phenyl-4-oxazolyl)propionyl]-1-naphthylmethyl]-2,4-thiazolidindion
s) 5-[4-[3-(5-Methyl-2ephenyl-4-oxazolyl)-1-hydroxypropyl]-1-naphthylmethyl]-2,4-thiazolidindion
t) 5-[4-[2-(5-Methyl-2-phenyl-4-oxazolyl)acetyl]-1-naphthylmethyl]-2,4-thiazolidindion
u) 5-[4-[2-(5-Methyl-2-phenyl-4-thiazolyl)ethoxy]-1-naphthylmethyl]-2,4-thiazolidindion
v) 5-[4-[2-(5-Methyl-2-phenyl-4-imidazolyl)ethoxy]-1-naphthylmethyl)- 2,4-thiazolidindion
sowie deren physiologisch verträgliche Salze.

3. 5-[4-[2-(5-Methyl-2-phenyl-4-oxazolyl)ethoxy]-7-benzothiophenmethyl]-2,4-thiazolidindion sowie dessen physiologisch verträgliche Salze.

4. Verfahren zur Herstellung von Verbindungen der Formel 1 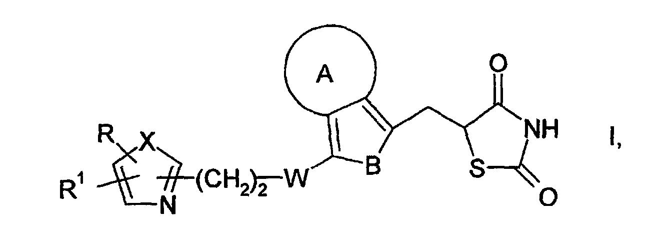 in der
A einen carbocyclischen Ring mit 5 oder 6 Kohlenstoffatomen oder einen heterocyclischen Ring mit 1 oder 2 Heteroatomen darstellt, wobei die Heteroatome Sauerstoff, Stickstoff oder Schwefel bedeuten, und die Heterocyden gegebenenfalls an einem oder mehreren Stickstoffatomen ein Sauerstoffatom tragen können,
B -CH=CH-, -N=CH-, -CH=N-,
W CH₂, O, CH (OH) oder CO,
x S, O oder NH,
R R Naphthyl, Pyridyl, Furyl, Thienyl oder Phenyl, das gegebenenfalls mit C₁-C₃ Alkyl, CF₃, C-C₃ Alkoxy, F, CI oder Brom mono- oder disubstituiert ist, und
R¹ Wasserstoff oder C₁-C₆ Alkyl
bedeutet, sowie deren Tautomere, Enantiomere, Diastereomere und physiologisch verträgliche Salze,
**dadurch gekennzeichnet, daß** man in an sich bekannter Weise
a) Verbindungen der allgemeinen Formel II 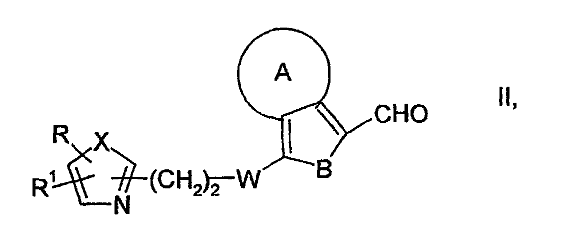 in der A, B, W, X, R und R¹ die oben angegebene Bedeutung haben, mit Thiazolidindion zu Verbindungen der allgemeinen Formel III 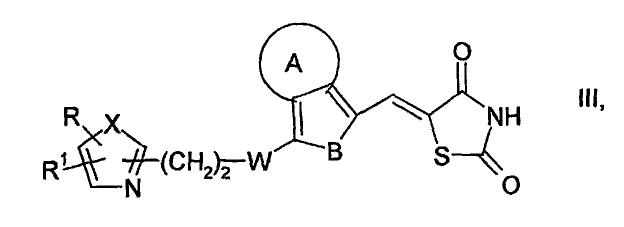 in der A, B, W, X, R und R¹ die oben angegebene Bedeutung haben, umsetzt und anschliessend durch Reduktion der Doppelbindung die Verbindungen der allgemeinen Formel I erhält, oder
b) Verbindungen der allgemeinen Formel IV 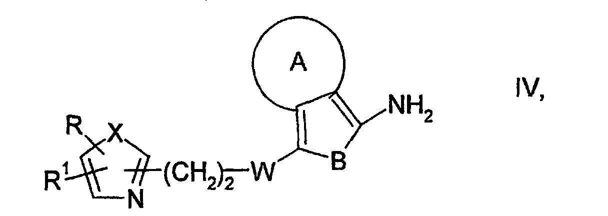 in der A, B, W, X, R und R¹ die oben angegebene Bedeutung haben, mit NaNO₂ in Gegenwart von Acrylester und HCl oder HBr zu Verbindungen der allgemeinen Formel V 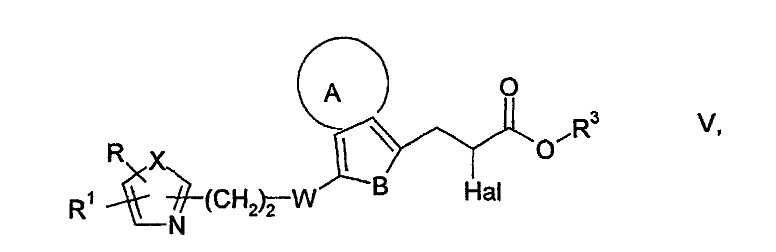 in der A, B, W, X, R und R¹ die oben angegebene Bedeutung haben, Hal für Chlor oder Brom steht und R³ ein C₁-C₆ -Alkylrest bedeutet, umsetzt und anschließend Verbindungen der allgemeinen Formel V mit Thioharnstoff zu Verbindungen der allgemeinen Formel VI 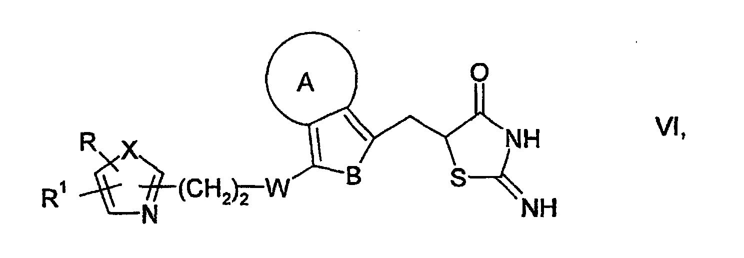 in der A, B, W, X, R und R¹ die oben angegebene Bedeutung haben, cyclisiert und durch Behandeln mit Säure in Verbindungen der allgemeinen Formel 1 umwandelt,
und anschließend die erhaltenen Verbindungen gewünschtenfalls in deren Tautomere, physiologisch verträgliche Salze sowie deren optische Isomeren überführt.

5. Arzneimittel, enthaltend mindestens eine Verbindung gemäss einem der Anprüche 1 - 3, neben üblichen Träger- und Hilfsstoffen.

6. Verwendung von Verbindungen gemäss einem der Ansprüche 1-3, zur Herstellung von Arzneimitteln zur Behandlung von Diabetes.

## Claims

1. Compounds of formula I 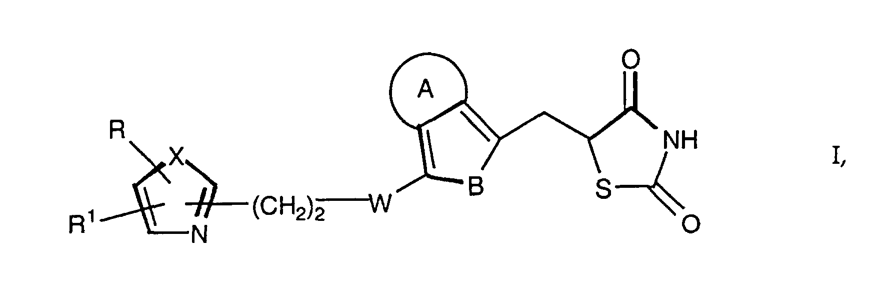 in which
A represents a carbocyclicring with 5 or 6 carbon atoms or a heterocyclic five or six ring with 1 or 2 hetero atoms, with the hetero atoms being oxygen, nitrogen or sulphur, and the heterocycles can, if desired, carry an oxygen atom on one or more nitrogen atoms,
B signifies -CH=CH-, -N=CH- or -CH=N-,
W signifies CH₂, O, CH(OH) or CO,
X signifies S, O or NH,
R signifies naphthyl, pyridyl, furyl, thienyl or phenyl which, if desired, is mono- or disubstituted with C₁-C₃ alkyl, CF₃, C₁-C₃ alkoxy, F, Cl or Br, and
R¹ signifies hydrogen or C₁-C₆ alkyl,
as well as their tautomers, enantiomers, diastereomers and physiologically tolerable salts.

2. A compound selected from the group
a) 5-[4-[2-(5-methyl-2-phenyl-4-oxazolyl)ethoxy]-1-naphthylmethyl]-2,4-thiazolidinedione
b) 5-[7-[2-(5-methyl-2-phenyl-4-oxazolyl)ethoxy]-4-indolylmethyl]-2,4-thiazolidinedione
c) 5- [7-[2-(5-methyl-2-phenyl-4-oxazolyl)ethoxy]-4-benzofuranylmethyl]-2,4-thiazolidinedione
d) 5-[7-[2-(5-methyl-2-phenyl-4-oxazolyl)ethoxy]-4-benzothiophenylmethyl]-2,4-hiazolidinedione
e) 5-(4-[2-(5-methyl-2-phenyl-4-oxazolyl)ethoxy]-7-indolylmethyl] -2,4-thiazolidinedione
f) 5-[4-[2-(5-methyl-2-phenyl-4-oxazolyl)ethoxy]-7-benzofuranylmethyl]-2,4-thiazolidinedione
g) 5-[4-(2-(5-methyl-2-phenyl-4-oxazolyl)ethoxy]-7-benzothiophenylmethyl]-2,4-thiazoldinedione
h) 5-[8-[2-(5-methyl-2-phenyl-4-oxazolyl)ethoxy]-5-quinolinylmethyl]-2,4-thiazolidinedione
i) 5- [8-[2-(5-methyl-2-phenyl-4-oxazolyl)ethoxy]-5-isoquinolinylmethyl]-2,4-thiazolidinedione
j) 5-[5-[2-(5-methyl-2-phenyl-4-oxazolyl)ethoxy]-8-isoquinolinylmethyl]-2,4-thiazolidinedione
k) 5-[5-[2-(5-methyl-2-phenyl-4-oxazolyl)ethoxy]-8-quinolinylmethyl]-2,4-thiazolidinedione
l) 5-[1-[2-(5-methyl-2-phenyl-4-oxazolyl)ethoxy] -4-isoquinolinylmethyl]-2,4-thiazolidinedione
m) 5- [4-[2-(5-methyl-2-phenyl-4-oxazolyl)ethoxy]-1-isoquinolinylmethyl] -2,4-thiazolidinedione
n) 5-[4-[2-[5-methyl-2-(4-methylphenyl)-4-oxazolyl)ethoxy]-1-naphthyl-methyl] -2,4-thiazolidinedione
o) 5-[4- [2-[5-methyl-2-(2-thienyl)-4-oxazolyl)ethoxy]-1-naphthylmethyl] -2,4-thiazolidinedione
p) 5-[4-[2-[5-methyl-2-(4-pyridyl)-4-oxazolyl)ethoxy]-1-naphthylmethyl]-2,4-thiazolidinedione
q) 5-[4-(5-methyl-2-phenyl-4-oxazolyl)methoxy]-1-naphthylmethyl]-2,4-thiazolidinedione
r) 5-[4-[3-(5-methyl-2-phenyl-4-oxazolyl)propionyl]-1-naphthylmethyl]-2,4-thiazolidinedione
s) 5-[4-[3-(5-methyl-2ephenyl-4-oxazolyl)-1-hydroxypropyl]-1-naphthyl-methyl] -2,4-thiazolidinedione
t) 5-[4-[2-(5-methyl-2-phenyl-4-oxazolyl)acetyl]-1-naphthylmethyl]-2,4-thiazolidinedione
u) 5-[4-[2-(5-methyl-2-phenyl-4-thiazolyl)ethoxy]-1-naphthylmethyl]-2,4-thiazolidinedione
v) 5-[4- [2-(5-methyl-2-phenyl-4-imidazolyl)ethoxy]-1-naphthylmethyl)-2,4-thiazolidinedione
as well as their physiologically compatible salts.

3. 5-[4-[2-(5-Methyl-2-phenyl-4-oxazolyl)ethoxy]-7-benzothiophenmethyl]-2,4-thiazolidinedione as well as its physiologically tolerable salts.

4. A process for the production of compounds of formula I 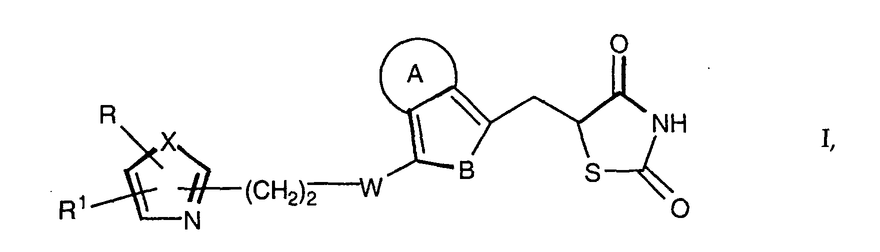 in which
A represents a carbocyclic ring with 5 or 6 carbon atoms or a heterocyclic ring with 1 or 2 hetero atoms, with the hetero atoms being oxygen, nitrogen or sulphur, and the heterocycles can, if desired, carry an oxygen atom on one or more nitrogen atoms,
B signifies -CH=CH-, -N=CH-, -CH=N-,
W signifies CH₂, O, CH(OH) or CO,
x signifies S, O or NH,
R signifies naphthyl, pyridyl, furyl, thienyl or phenyl which, if desired, is mono- or disubstituted with C₁-C₃ alkyl, CF₃, C₁-C₃ alkoxy, F, Cl or Br, and
R¹ signifies hydrogen or C₁-C₆ alkyl,
as well as their tautomers, enantiomers, diastereomers and physiologically tolerable salts, **characterized in that** in a manner known per se
a) compounds of general formula II 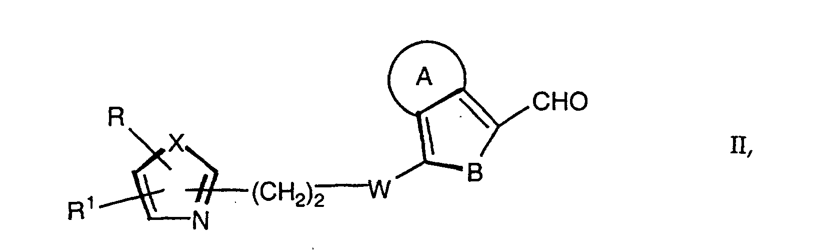 in which A, B, W, X, R and R¹ have the significance given above,
are reacted with thiazolidinedione to give compounds of general formula III 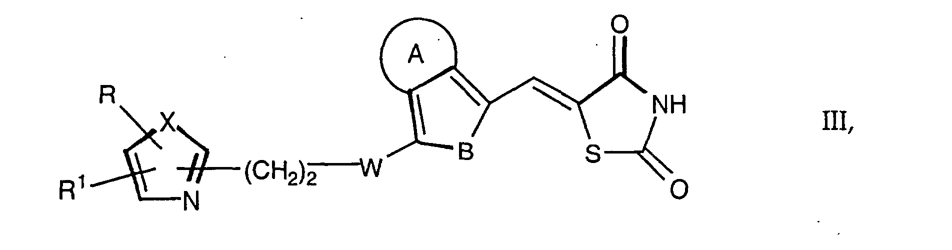 in which A, B, W, X, R and R¹ have the significance given above,
and subsequently the compounds of general formula I are obtained by reduction of the double bond, or
b) compounds of general formula IV 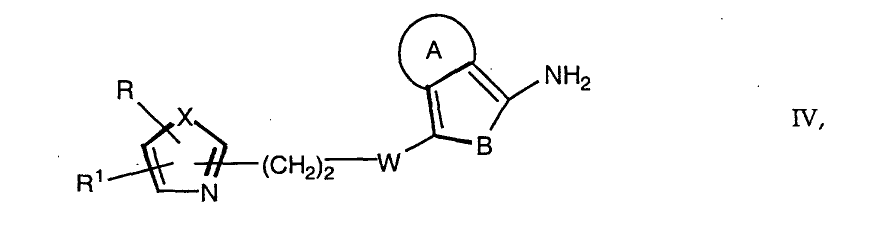 in which A, B, W, X, R and R¹ have the significance given above,
are reacted with NaNO₂ in the presence of acrylic ester and HCl or HBr to give compounds of general formula V 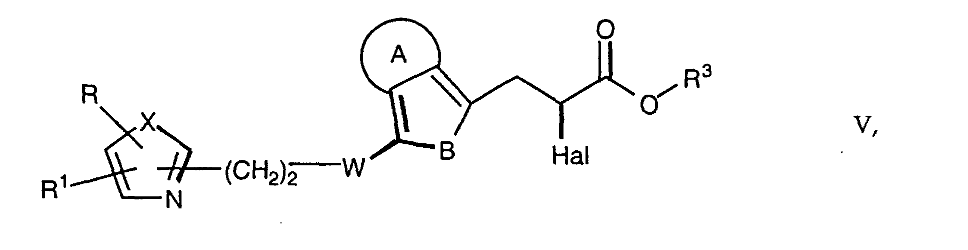 in which A, B, W, X, R and R¹ have the significance given above, Hal stands for chlorine or bromine and R³ signifies a C₁-C₆-alkyl residue,
and subsequently compounds of general formula V are cyclized with thiourea to give compounds of general formula VI 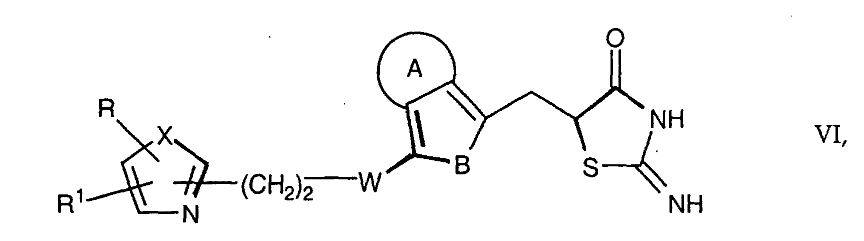 in which A, B, W, X, R and R¹ have the significance given above,
and converted into compounds of general formula I by treatment with acid,
and subsequently the compounds obtained are, if desired, converted into their tautomers, physiologially tolerable salts as well as their optical isomers.

5. A medicament containing at least one compound in accordance with any one of claims 1-3 in addition to usual carriers and auxiliary substances.

6. The use of compounds in accordance with any one of claims 1-3 for the production of medicaments for the treatment of diabetes.

## Revendications

1. Composés de formule I 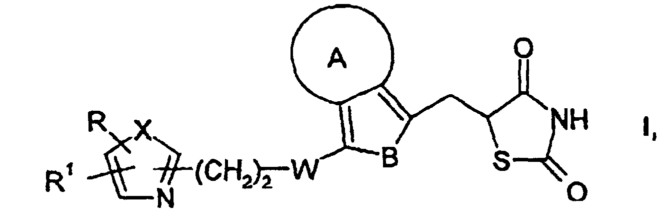 dans laquelle
A est un noyau carbocyclique ayant 5 ou 6 atomes de carbone ou un noyau hétérocyclique à 5 ou 6 chaînons ayant 1 ou 2 hétéroatomes, les hétéroatomes étant l'oxygène, l'azote ou le soufre, et les radicaux hétérocycliques pouvant éventuellement porter un atome d'oxygène sur un ou plusieurs atomes d'azote,
B est -CH=CH-, -N=CH- ou -CH=N-,
W est CH₂, O, CH(OH) ou CO,
X est S, O ou NH,
R est un radical naphtyle, pyridyle, furyle, thiényle ou phényle, qui est éventuellement mono- ou disubstitué par des substituants alkyle en C₁-C₃, CF₃, alcoxy en C₁-C₃, F, Cl ou bromo, et
R¹ est un hydrogène ou un groupe alkyle en C₁-C₆,
ainsi que leurs tautomères, énantiomères, diastéréoisomères et sels compatibles d'un point de vue physiologique.

2. Composé choisi dans l'ensemble suivant :
a) 5-[4-[2-(5-méthyl-2-phényl-4-oxazolyl)éthoxy]-1-naphtylméthyl]-2,4-thiazolidinedione,
b) 5-[7-[2-(5-méthyl-2-phényl-4-oxazolyl)éthoxy]-4-indolylméthyl]-2,4-thiazolidinedione,
c) 5-[7-[2-(5-méthyl-2-phényl-4-oxazolyl)éthoxy]-4-benzofurannylméthyl]-2,4-thiazolidinedione,
d) 5-[7-[2-(5-méthyl-2-phényl-4-oxazolyl)éthoxy]-4-benzothiophénylméthyl]-2,4-thiazolidinedione,
e) 5-[4-[2-(5-méthyl-2-phényl-4-oxazolyl)éthoxy]-7-indolylméthyl]-2,4-thiazolidinedione,
f) 5-[4-[2-(5-méthyl-2-phényl-4-oxazolyl)éthoxy]-7-benzofurannylméthyl]-2,4-thiazolidinedione,
g) 5-[4-[2-(5-méthyl-2-phényl-4-oxazolyl)éthoxy]-7-benzothiophénylméthyl]-2,4-thiazolidinedione,
h) 5-[8-[2-(5-méthyl-2-phényl-4-oxazolyl)éthoxy]-5-quinolinylméthyl]-2,4-thiazolidinedione,
i) 5-[8-[2-(5-méthyl-2-phényl-4-oxazolyl)éthoxy]-5-isoquinolinylméthyl]-2,4-thiazolidinedione,
j) 5-[5-[2-(5-méthyl-2-phényl-4-oxazolyl)éthoxy]-8-isoquinolinylméthyl]-2,4-thiazolidinedione,
k) 5-[5-[2-(5-méthyl-2-phényl-4-oxazolyl)éthoxy]-8-quinolinylméthyl)-2,4-thiazolidinedione,
l) 5-[1-[2-(5-méthyl-2-phényl-4-oxazolyl)éthoxy]-4-isoquinolinylméthyl]-2,4-thiazolidinedione,
m) 5-[4-[2-(5-méthyl-2-phényl-4-oxazolyl)éthoxy]-1-isoquinolinylméthyl]-2,4-thiazolidinedione,
n) 5-[4-[2-[5-méthyl-(4-méthylphényl)-4-oxazolyl]éthoxy]-1-naphtylméthyl]-2,4-thiazolidinedione,
o) 5-[4-[2-[5-méthyl-2-(2-thiényl)-4-oxazolyl]éthoxy]-1-naphtylméthyl]-2,4-thiazolidinedione,
p) 5-[4-[2-[5-méthyl-2-(4-pyridyl)-4-oxazolyl]éthoxy]-1-naphtylméthyl]-2,4-thiazolidinedione,
q) 5-[4-[2-(5-méthyl-2-phényl-4-oxazolyl)méthoxy]-1-naphtylméthyl]-2,4-thiazolidinedione,
r) 5-[4-[3-(5-méthyl-2-phényl-4-oxazolyl)propionyl]-1-naphtylméthyl]-2,4-thiazolidinedione,
s) 5-[4-[3-(5-méthyl-2-phényl-4-oxazolyl)-1-hydroxypropyl]-1-naphtylméthyl]-2,4-thiazolidinedione,
t) 5-[4-[2-(5-méthyl-2-phényl-4-oxazolyl)acétyl]-1-naphtylméthyl]-2,4-thiazolidinedione,
u) 5-[4-[2-(5-méthyl-2-phényl-4-thiazolyl)éthoxy]-1-naphtylméthyl]-2,4-thiazolidinedione,
v) 5-[4-[2-(5-méthyl-2-phényl-4-imidazolyl)éthoxy]-1-naphtylméthyl]-2,4-thiazolidinedione,
ainsi que ses sels acceptables d'un point de vue physiologique.

3. 5-[4-[2-(5-méthyl-2-phényl-4-oxazolyl)éthoxy]-7-benzothiophéneméthyl]-2,4-thiazolidinedione, ainsi que ses sels acceptables d'un point de vue physiologique.

4. Procédé pour préparer des composés de formule I 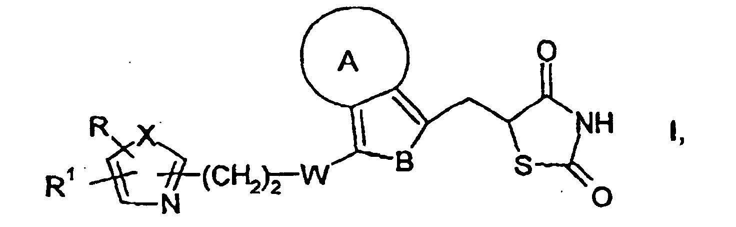 dans laquelle
A est un noyau carbocyclique ayant 5 ou 6 atomes de carbone ou un noyau hétérocyclique ayant 1 ou 2 hétéroatomes, les hétéroatomes étant l'oxygène, l'azote ou le soufre, et les radicaux hétérocycliques pouvant éventuellement porter un atome d'oxygène sur un ou plusieurs atonies d'azote,
B est -CH=CH-, -N=CH- ou -CH=N-,
W est CH₂, O, CH(OH) ou CO,
X est S, O ou NH,
R est un radical naphtyle, pyridyle, furyle, thiényle ou phényle, qui est éventuellement mono- ou disubstitué par des substituants alkyle en C₁-C₃, CF₃, alcoxy en C₁-C₃, F, Cl ou bromo, et
R¹ est un atome d'hydrogène ou un groupe alkyle en-C₁-C₆,
ainsi que leurs tautomères, énantiomères, diastéréoisomères et sels acceptables d'un point de vue physiologique,
**caractérisé en ce que**, d'une manière connue en soi,
a) on fait réagir des composés de formule générale II 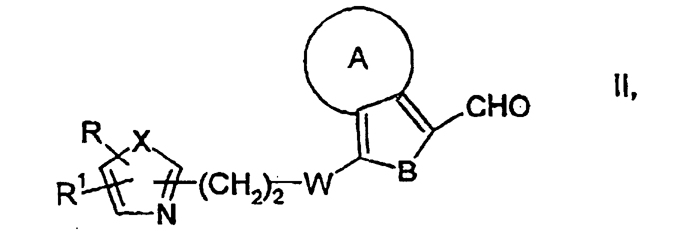 dans laquelle A, B, W, X, R et R¹ ont les significations données ci-dessus, avec de la thiazolidinedione, pour obtenir des composés de formule générale III 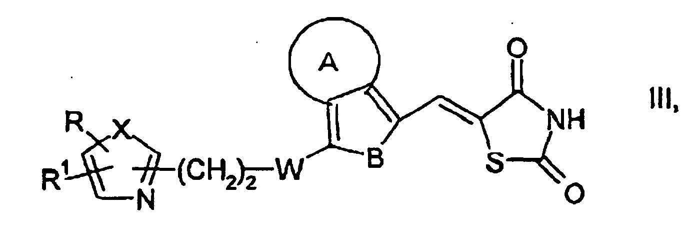 dans laquelle A, B, W, X, R et R¹ ont les significations ci-dessus, puis, par réduction de la double liaison, on obtient les composés de formule générale I, ou bien
b) on fait réagir des composés de formule générale IV 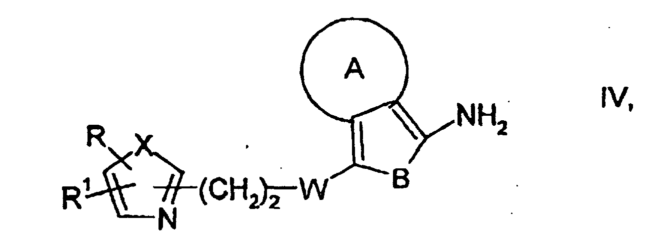
dans laquelle A, B, W, X, R et R¹ ont les significations données ci-dessus, avec du NaNO₂ en présence d'un acrylate et de HCl ou de HBr, pour obtenir des composés de formule générale V 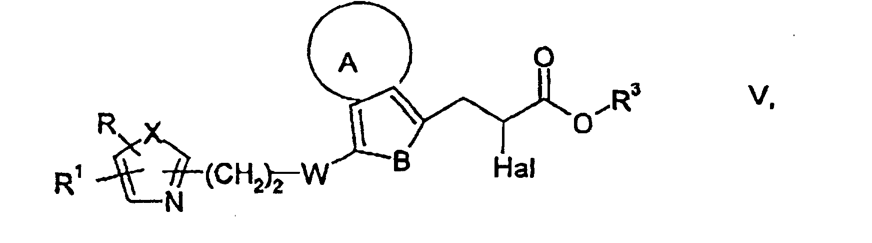 dans laquelle A, B, W, X, R et R¹ ont les significations données ci-dessus, Hal représente le chlore ou le brome et R³ est un radical alkyle en C₁-C₆, puis on cyclise les composés de formule générale V à l'aide de thiourée pour obtenir des composés ayant la formule générale VI 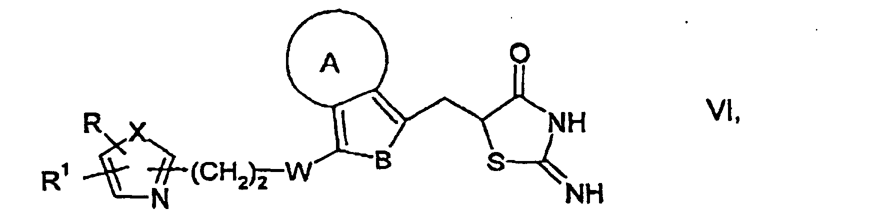 dans laquelle A, B, W, X, R et R¹ ont les significations données ci-dessus, et on les convertit par traitement avec un acide en des composés de formule générale I,
puis, si on le souhaite, on convertit les composés obtenus en leurs tautomères, sels acceptables d'un point de vue physiologique, ou en leurs isomères optiques.

5. Médicament contenant au moins un composé selon l'une des revendications 1 à 3, en plus d'excipients et adjuvants usuels.

6. Utilisation de composés selon l'une des revendications 1 à 3 pour préparer des médicaments destinés au traitement du diabète.
